# EUROPEAN PATENT SPECIFICATION

(11) **EP 1 349 516 B1**
(45) Date of publication and mention of the grant of the patent: **13.09.2006**
(21) Application number: 01986149.1
(22) Date of filing: 12.12.2001
(51) Int. Cl.: A61F 2/84

(54) **LUBRICANT FILLER PORT FOR STENT DELIVERY SYSTEM**
SCHMIERMITTELEINFÜLLÖFFNUNG FÜR EIN STENTIMPLANTATIONSSYSTEM
ORIFICE DE REMPLISSAGE DE LUBRIFIANT POUR UN SYSTÈME DE MISE EN PLACE D'UN STENT

(30) Priority: 12.12.2000 US 735325
(43) Date of publication of application: 08.10.2003
(73) Proprietor: Boston Scientific Limited, St. Michael, Barbados, West Indies (BB)
(72) Inventor: HACKETT, Steven, S., Maple Grove, MN 55369 (US); DICAPRIO, Fernando, St. Paul, MN 55105 (US); TAYLOR, Kyle, P., Brooklyn Park, MN 55445 (US); SEPPALA, Jan, D., Maple Grove, MN 55369 (US)
(74) Representative: Hauck, Graalfs, Wehnert, Döring, Siemons
(86) International application number: PCT/US2001/047972
(87) International publication number: WO 2002/047752

(56) References cited:
- WO-A-00/76425
- WO-A-01/78628
- US-A- 4 950 227
- US-A- 5 403 341
- US-A- 6 063 112

## Description

### BACKGROUND OF THE INVENTION

### Field of the Invention:

The invention relates to a medical device delivery system, namely catheter mounted stent delivery system. More particularly, the present invention is directed to socks or sleeves used to retain a stent on a stent delivery catheter. The present invention provides for one or more stent end retaining sleeves having a single lubricant filler port which may be used to apply lubricant to the balloon cone and/or waist without excess wicking of lubricant onto the balloon body and stent.

### Description Of The Related Art:

Stents and stent delivery assemblies are utilized in a number of medical procedures and situations, and as such their structure and function are well known. A stent is a generally cylindrical prosthesis introduced via a catheter into a lumen of a body vessel in a configuration having a generally reduced diameter and then expanded to the diameter of the vessel. In its expanded configuration, the stent supports and reinforces the vessel walls while maintaining the vessel in an open, unobstructed condition.

Both self-expanding and inflation expandable stents are well known and widely available in a variety of designs and configurations. Self-expanding stents must be maintained under a contained sheath or sleeve(s) in order to maintain their reduced diameter configuration during delivery of the stent to its deployment site. Inflation expandable stents are crimped to their reduced diameter about the delivery catheter, then maneuvered to the deployment site and expanded to the vessel diameter by fluid inflation of a balloon positioned between the stent and the delivery catheter. The present invention is particularly concerned with delivery and deployment of inflation expandable stents, although it is generally applicable to self-expanding stents when used with one or more stent retaining sheaths.

In advancing an inflation expandable stent through a body vessel to the deployment site, there are a number of important considerations. The stent must be able to securely maintain its axial position on the delivery catheter without translocating proximally or distally and especially without becoming separated from the catheter. The stent, particularly its distal and proximal ends, must be protected to prevent distortion of the stent and to prevent abrasion and/or reduce trauma of the vessel walls.

Inflation expandable stent delivery and deployment assemblies are known which utilize restraining means that overlie the stent during delivery. U.S. Patent No. 4,950,227 to Savin et al., relates to an inflation expandable stent delivery system in which a sleeve overlaps the distal or proximal margin (or both) of the stent during delivery. During inflation of the stent at the deployment site, the stent margins are freed of the protective sleeve(s). U.S. Patent 5,403,341 to Solar, relates to a stent delivery and deployment assembly which uses retaining sheaths positioned about opposite ends of the compressed stent. The retaining sheaths of Solar are adapted to tear under pressure as the stent is radially expanded, thus releasing the stent from engagement with the sheaths. U.S. Patent No. 5,108,416 to Ryan et al., describes a stent introducer system which uses one or two flexible end caps and an annular socket surrounding the balloon to position the stent during introduction to the deployment site.

Document WO 01/78628, part of the state of the art according to Article 54(3) EPC, describes, equally to documents US 4850227 and US 5403341, a stent delivery system according to the preamble of claim 1 of the present invention. In WO 01178628 the stent retaining sleeves have a plurality of through-holes to achieve reduced radial strength of the sleeve. The through-holes may be used to apply lubricant to a part of the balloon. A common problem which occurs in catheter assemblies is friction or adhesion between various parts which periodically come into contact with one another during the medical procedure. For instance, friction can occur between the guide catheter and guide wire, between the introducer sheath and the guide catheter, or between the guide catheter and the balloon catheter, for instance, and may increase the difficulty of insertion, cause loss of catheter placement, and result in discomfort to the patient or damage to the vasculature. In catheters equipped with stent retaining socks or sleeves, friction between the balloon and sleeve, and/or the stent and sleeve may also cause retraction of the sleeves to be made more difficult. It is therefore desirable to reduce the friction due to the sliding between the various parts of the catheter assemblies.

The materials from which catheters are produced are typically polymeric or metallic in nature, and in general are inherently non-lubricious. When these non-lubricious materials come into contact, friction occurs. Medical device manufacturers have used various approaches to reduce the co-efficient of friction between these surfaces.

Lubricants of many types have been used in conjunction with balloon catheters. Both hydrophilic and hydrophobic coatings and lubricants are well known in the catheter art. The present invention may be used in conjunction with any type of lubricious substance suitable for use with a stent delivery catheter, and is further directed to the application of the lubricious substance to the surface of a balloon cone and/or waist subsequent to stent mounting and sleeve placement onto the catheter.

The present invention is directed to a method of applying any type of lubricious substance to the surface of a balloon cone, even after the stent and stent retaining sleeves are mounted to the catheter.

The problem is solved by a stent delivery system according to claim 1.

### BRIEF SUMMARY OF THE INVENTION

The present invention is directed to providing a stent retaining sleeve with a single lubricant application port. The lubricant port allows lubricant application to the balloon cone after the sleeve is mounted onto the stent delivery catheter. The port may be positioned along the portion of the waist and/or the cone portion of the sleeve. Where the port is located on the cone portion, or extend to the cone portion, the port is constructed to provide controlled sleeve fracture to assist in sleeve retraction. The sleeve may be used singly or in pairs with either self-expanding or balloon expandable stents. In the case of a self-expanding stent, one or more sleeves may be utilized in conjunction with one or more retractable sheaths. The sleeve(s) may be provided in a variety of lengths to provide partial to full stent coverage. Other inventive aspects and embodiments of the present end retaining sleeves will be made apparent below.

### BRIEF DESCRIPTION OF THE SEVERAL VIEWS OF THE DRAWINGS

A detailed description of the invention is hereafter described with specific reference being made to the drawings in which:
- Fig. 1: is a side perspective view of a first embodiment of the invention;
- Fig. 2: is a side perspective view of a second embodiment of the invention;
- Fig. 3: is a side perspective view of an embodiment not according to the present invention;
- Fig. 4: is a side perspective view of an embodiment not according to the present invention;
- Fig. 5: is a side perspective view of a third embodiment of the invention;
- Fig. 6: is a side perspective view of a fourth embodiment of the invention;
- Fig. 7: is a side perspective view of the embodiment shown in Fig. 6 as may be seen during stent expansion;
- Fig. 8: is a side perspective view of the embodiment shown in Fig. 7 as may be seen during sleeve retraction; and
- FIG. 9: is a side perspective view of an alternative embodiment not according to the present invention.

### DETAILED DESCRIPTION OF THE INVENTION

While this invention may be embodied in many different forms, there are shown in the drawings and described in detail herein specific preferred embodiments of the invention. The present disclosure is an exemplification of the principles of the invention and is not intended to limit the invention to the particular embodiments illustrated.

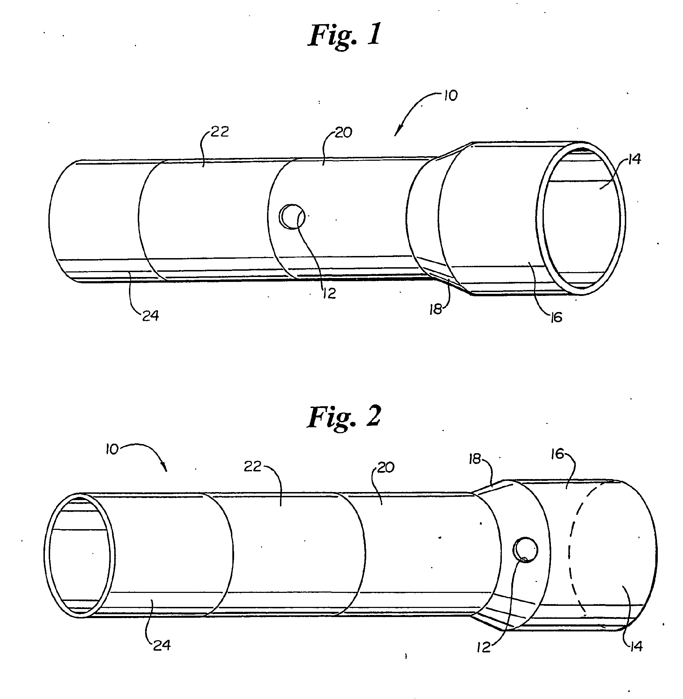

As may be seen in FIG. 1, the present invention may be embodied in an elastomeric sock or sleeve 10 having a single port 12 which may be utilized to inject or otherwise place a lubricious substance (not shown) on to the inside surface 14 of the sleeve 10. Sleeves such as sleeve 10 presently described, may be characterized as having a stent overlaying portion 16, a cone portion 18, a cone waist transition portion 20, a waist portion 22 and a balloon end portion 24.

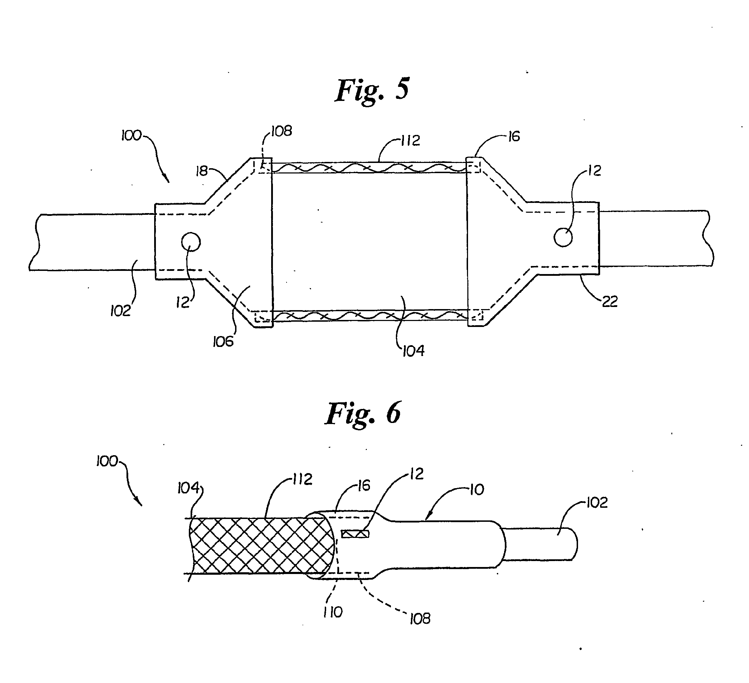

The port 12 may be located anywhere on the sleeve 10. However, as may be seen in the embodiment shown in FIG. 1, the port 12 is positioned immediately adjacent to the waist portion 22, and through a portion of the cone waist transition portion 20. Because the waist portion 22 may be engaged to a catheter shaft, the position of the port 12 in the embodiment shown in FIG. 1, ensures that a lubricant inserted therethrough will have maximum coverage of the inside surface 14 of the sleeve 10 where it overlays a respective balloon cone 106 and stent end 108, such as may best be seen in FIG. 5.

When the sleeve 10 is used in conjunction with a stent delivery catheter 100 such as may be seen in FIG. 5, the port 12 is preferably located immediately adjacent to the section of the sleeve 10, such as the waist portion 22 where the sleeve 10 is engaged or bonded to the catheter shaft 102. Where the balloon 104 is integral with the shaft 10 the port 12 may be adjacent to the balloon end portion 24.

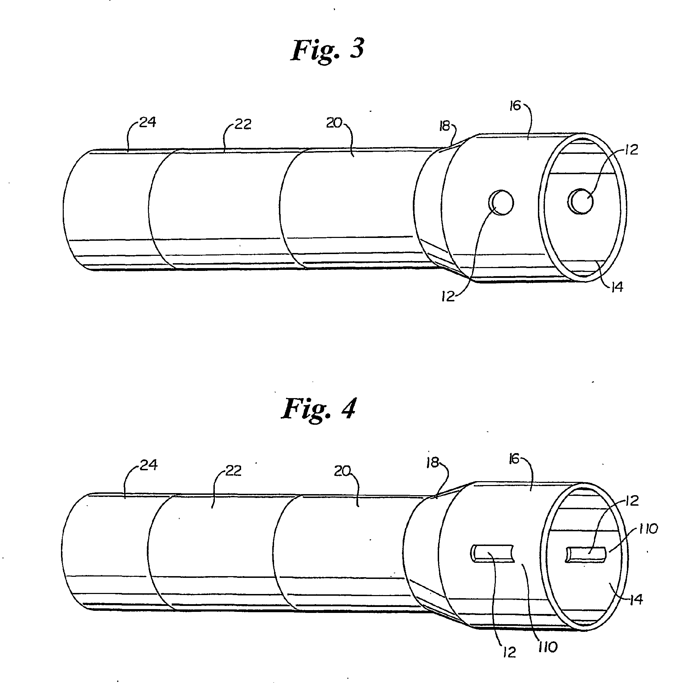

While the relative positions of ports 12 shown in FIG. 1 and FIG. 5 may be preferred. The ports 12 may be placed in an infinite variety of positions and combinations. It should also be noted that while a single port 12 may be used, two or more opposingly positioned ports 12 may be utilized such as are illustrated in FIG. 3. In addition a variety of ports, which vary in number, shape and position may also be utilized.

Some examples of alternative port placement are provided as follows: As may be seen in FIG. 2, the port 12 is defined by the cone portion 18. Such port 12 placement may be desired to help ensure lubricant application to the balloon cone 106 (such as may be seen in FIG. 5) which may lie thereunder.

FIG. 3 shows an embodiment of the sleeve 10 not according to the present invention wherein the ports 12 are defined by the stent overlaying portion 16. Such port 12 placement may be desired to help ensure lubricant application to the stent end 108 (such as may be seen in FIG. 5) which may lie thereunder.

As previously indicated, port(s) 12 may have a variety of shapes and sizes as well as positions. As may be seen in FIG. 4, a pair of ports 12 are shown having a rectangular configuration, however any shapes may also be used. In the embodiment shown the ports 12 are defined by the stent overlaying portion 16. The ports 12 may be configured to fracture or otherwise tear the thin portion of sleeve material 110 which defines at least one side of the port. When a sleeve 10 is used in conjunction with a stent delivery catheter 100 such as shown in FIG. 6, the ports 12 may be positioned in whole or in part over the stent ends 108.

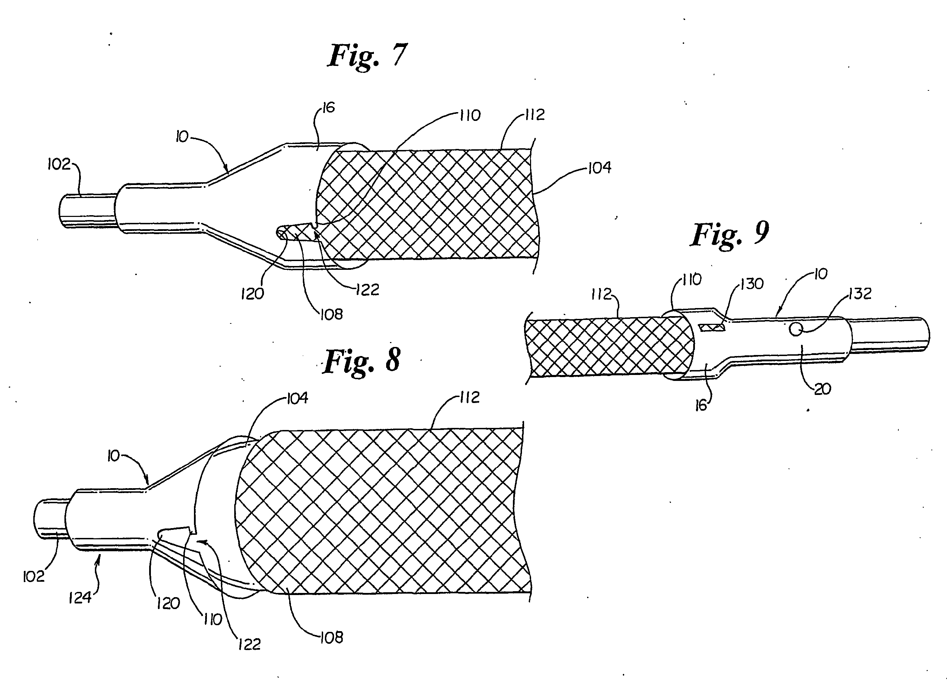

The balloon 104 and stent 112 may be expanded from an unexpanded state, such as may be seen in FIG. 6, to an expanded state, such as may be seen in FIG. 8. During stent 112 expansion, one or more portions of the sleeve 10, most notably the stent overlaying portion 16 may also have a predetermined amount of outward pressure exerted on it. The thin portion of material 110 is constructed and arranged to rupture when a predetermined amount of outwardly acting force is exerted against at least a portion of the sleeve 10 such as is exerted against the sleeve 10 during stent 112 expansion, such as may be seen in FIG. 7.

As is shown in FIG. 7, during expansion the rupture of the thin portion of material 110 result in a slot 120 having an opening 122. The opening 122 of the slot 120 may be characterized as a break in the sleeve 10 which allows at least the stent overlaying portion 16 to split thereby providing for a sudden increase in stent overlaying portion 16 diameter and a resulting reduction in frictional engagement and radial compression which would otherwise be provided by the stent overlaying portion 16 of the sleeve 10 against the expanding stent 112. This reduces radial sleeve strength thereby allowing the sleeve 10 to more readily retract off of the stent ends 108, such as may best be seen in Fig. 8, thereby freeing the stent 112 from under the sleeve 10 as a result of expansion.

As may also be seen in Fig. 8, after the material 110 breaks or ruptures in the manner described above, the sleeve 10 is constructed and arranged to retract off of the stent ends 108 in the manner shown and indicated by reference arrow 124.

The sleeve 10 may be retracted off of the stent 112 in the folding configuration indicated by arrow 124 or the stent may roll up on itself along the catheter shaft 102. It should be noted however, that any type of retraction mode may be attributed to the sleeves 10, in addition or instead of the configuration shown and described.

Turning to fig. 9, an alternative embodiment not according to the present invention is illustrated wherein two different types of lubricant injection ports are positioned on a stent retaining sleeve 10. As shown a first tear-away port 130 is defined by the thin material 110 and the stent overlaying portion 16. A second port 132 is defined by the cone waist transition portion 20. By providing the sleeve 10 with multiple lubricant filler ports the inside surface 14 of the sleeve 10 may be more throughly lubricated. In addition, the tear-away port 1342 helps to provide controlled sleeve rupture for controlled stent 112 release such as previously described.

The above examples and disclosure are intended to be illustrative and not exhaustive. These examples and description will suggest many variations and alternatives to one of ordinary skill in this art, within the scope of the following claims.

## Claims

1. A stent delivery system comprising:
- a stent delivery catheter (100), the stent delivery catheter having a balloon (104) mounted thereon, the balloon having an unexpanded position and an expanded position, the balloon having at least one waist portion, at least one cone portion (106) and at least one body portion;
- a stent (112) disposed about the at least one body portion, the stent having an expanded state and an unexpanded state;
- at least one sleeve (10), the at least one sleeve having a waist overlay portion (22), a cone overlay portion (18), and a stent overlay portion (16), when the stent is in the unexpanded state the waist overlay portion being engaged to at least a portion of the at least one waist portion and the cone overlay portion being disposed about the at least one cone portion and the stent overlay portion being disposed about at least a portion of the stent,
**characterized in that**
- the at least one sleeve has a single lubricant application port (12), the at least one lubricant application port defining an opening through the at least one sleeve, the opening constructed and arranged to allow a lubricious substance to reach a surface of a portion of the balloon.

2. The stent delivery system of claim 1 wherein the lubricant application port (12) is at least partially defined by the waist overlay portion.

3. The stent delivery system of claim 1 wherein the lubricant application port (12) is at least partially defined by the cone overlay portion (18).

4. The stent delivery system of one of the claims 1 to 3, wherein the lubricant application port (12) is a tear-away lubricant application port at least partially defined by the portion of the stent overlay portion (16).

5. The stent delivery system of claim 4, wherein the at least one sleeve (10) is constructed and arranged to retract off of a stent subsequent to the fracture of the portion of the stent overlay portion.

## Patentansprüche

1. Ein Stentimplantationssystem, das umfasst:
- einen Stenttransportkatheter (100), wobei der Stenttransportkatheter einen auf ihm montierten Ballon (104) besitzt, der Ballon eine nicht expandierte Stellung und eine expandierte Stellung besitzt und der Ballon zumindest ein Mittelstück, zumindest ein Konusteil (106) und zumindest ein Körperteil besitzt;
- einen Stent (112), der um das zumindest eine Körperteil herum angeordnet ist, wobei der Stent einen expandierten Zustand und einen nicht expandierten Zustand besitzt;
- zumindest eine Manschette (10), wobei die zumindest eine Manschette ein mittelstücküberlagemdes Teil (22), ein konusteilüberlagerndes Teil (18) und ein stentüberlagemdes Teil (16) besitzt, wobei, wenn der Stent sich in dem nicht expandierten Zustand befindet, das mittelstücküberlagernde Teil mit zumindest einem Teil des zumindest einen Mittelstücks verbunden ist, und das konusteilüberlagernde Teil um das zumindest eine Konusteil herum angeordnet ist, und das stentüberlagemde Teil um zumindest einen Teil des Stents herum angeordnet ist,
**dadurch gekennzeichnet, dass**
- die zumindest eine Manschette eine einzelne Schmiermitteleinfüllöffnung (12) besitzt, wobei die zumindest eine Schmiermitteleinfüllöffnung eine Öffnung durch die zumindest eine Manschette hindurch festlegt, und die Öffnung konstruiert und angeordnet ist, um es einer gleitenden Substanz zu ermöglichen, eine Oberfläche eines Teils des Ballons zu erreichen.

2. Das Stentimplantationssystem gemäß Anspruch 1, wobei die Schmiermitteleinfüllöffnung (12) zumindest teilweise von dem mittelstücküberlagemden Teil begrenzt ist.

3. Das Stentimplantationssystem gemäß Anspruch 1, wobei die Schmiermitteleinfüllöffnung (12) zumindest teilweise vom konusteilüberlagernden Teil (18) begrenzt ist.

4. Das Stentimplantationssystem gemäß einem der Ansprüche 1 bis 3 wobei die Schmiermitteleinfüllöffnung (12) eine Abrissschmiermitteleinfüllöffnung darstellt, die zumindest teilweise von einem Teil des stentüberlagemden Teils (16) begrenzt ist.

5. Das Stentimplantationssystem gemäß Anspruch 4, wobei die zumindest eine Manschette (10) konstruiert und angeordnet ist, um sich von einem Stent zurückzuziehen, im Anschluss an den Bruch eines Teils des stentüberlagernden Teils.

## Revendications

1. Système de mise en place de stent comprenant :
- un cathéter de mise en place de stent (100), le cathéter de mise en place de stent comportant un ballon (104) monté sur celui-ci, le ballon ayant une position non expansée et une position expansée, le ballon comportant au moins une partie de taille, au moins une partie de cône (106) et au moins une partie de corps ;
- un stent (112) disposé autour d'au moins une partie de corps, le stent ayant un état expansé et un état non expansé ;
- au moins un manchon (10), le au moins un manchon ayant une partie superposée à la taille (22), une partie superposée au cône (18) et une partie superposée au stent (16), lorsque le stent est dans l'état non expansé la partie superposée à la taille étant engagée avec au moins une partie de la au moins une partie de taille et la partie superposée au cône étant disposée autour de la au moins une partie de cône et la partie superposée au stent étant disposée autour d'au moins une partie du stent,
**caractérisé en ce que**
- le au moins un manchon comporte un orifice d'application de lubrifiant unique (12), le au moins un orifice d'application de lubrifiant définissant une ouverture à travers le au moins un manchon, l'ouverture constituée et agencée pour permettre à une substance lubrifiante d'atteindre une surface d'une partie du ballon.

2. Système de mise en place de stent selon la revendication 1, dans lequel l'orifice d'application de lubrifiant (12) est au moins partiellement défini par la partie superposée à la taille.

3. Système de mise en place de stent selon la revendication 1, dans lequel l'orifice d'application de lubrifiant (12) est au moins partiellement défini par la partie superposée au cône (18).

4. Système de mise en place de stent selon l'une des revendications 1 à 3, dans lequel l'orifice d'application de lubrifiant (12) est un orifice d'application de lubrifiant déchirable défini au moins partiellement par la partie de la partie superposée au stent (16).

5. Système de mise en place de stent selon la revendication 4, dans lequel le au moins un manchon (10) est constitué et agencé pour se rétracter d'un stent après la fracture d'une partie de la partie superposée au stent.
